(19) Europäisches Patentamt
European Patent Office
Office européen des brevets

(11) **EP 3 045 438 B1**

(12) **EUROPÄISCHE PATENTSCHRIFT**

(45) Veröffentlichungstag und Bekanntmachung des Hinweises auf die Patenterteilung:
**29.11.2017 Patentblatt 2017/48**

(51) Int Cl.:
***C07C 2/10*** (2006.01)   ***C07C 7/04*** (2006.01)
***C07C 11/08*** (2006.01)   ***C07C 11/02*** (2006.01)
***C07C 11/107*** (2006.01)

(21) Anmeldenummer: **15151624.2**

(22) Anmeldetag: **19.01.2015**

(54) **Kombinierte Herstellung von zumindest Buten und Octen aus Ethen**

Combined production of at least butene and octene from ethene

Fabrication combinée d'au moins de butène et d'octène à partir d'éthylène

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**20.07.2016 Patentblatt 2016/29**

(73) Patentinhaber: **Evonik Degussa GmbH**
**45128 Essen (DE)**

(72) Erfinder:
• **Stochniol, Guido**
  **45721 Haltern am See (DE)**
• **Peitz, Stephan**
  **45739 Oer-Erkenschwick (DE)**
• **Maschmeyer, Dietrich**
  **45657 Recklinghausen (DE)**
• **Reeker, Helene**
  **44227 Dortmund (DE)**
• **Schallenberg, Jörg**
  **46286 Dorsten (DE)**

(56) Entgegenhaltungen:
**JP-A- 2003 326 169   US-A1- 2013 158 321**

**Beschreibung**

Die Erfindung befasst sich mit der kombinierten Herstellung von zumindest Buten und Octen aus Ethen.

Kohlenwasserstoffe sind chemische Verbindungen, die ausschließlich aus Kohlenstoff C und Wasserstoff H bestehen. Alkene (synonym: Olefine) sind Kohlenwasserstoffe, die eine C=C Doppelbindung im Molekül aufweisen. Alkane (synonym: Paraffine) sind dagegen Kohlenwasserstoffe, die nur Einfachbindungen aufweisen. Sie werden deswegen auch als gesättigt bezeichnet.

In der organischen Chemie werden Kohlenwasserstoffe häufig nach der Anzahl ihrer Kohlenstoffatome pro Molekül bezeichnet, indem der jeweiligen Substanzklasse das Präfix $C_n$ vorangestellt wird. Dabei bezeichnet n die jeweilige Anzahl der Kohlenstoffatome in einem Molekül. So sind $C_4$-Olefine zu verstehen als Substanzen der Klasse der Alkene mit vier Kohlenstoffatomen. $C_8$-Olefine weisen dementsprechend acht Kohlenstoffatome pro Molekül auf. Soweit im Folgenden das Präfix $C_{n+}$ verwendet wird, ist von einer Substanzklasse die Rede, die mehr als n Kohlenstoffatome pro Molekül aufweist. Ein $C_{4+}$-Olefin hat demnach mindestens fünf Kohlenstoffatome.

Das einfachste Olefin ist Ethen (Ethylen). Es weist zwei Kohlenstoffatome auf. Ethen stellt eine bedeutsame Grundchemikalie dar und wird daher in großen Mengen hergestellt. Dies geschieht meist durch Dampfspaltung von Naphtha. Darüber hinaus kann es durch Dehydrierung von Ethan gewonnen werden, welches wiederum ein Bestandteil des Erdgases ist. Aufgrund zunehmender Erschließung von unkonventionellen Erdgasquellen und abnehmender Erdöl-Förderung nimmt der Anteil an auf Erdgas basierendem Ethen stetig zu.

Zu den $C_4$- Olefinen gehören die vier isomeren Stoffe 1-Buten, cis-2-Buten, trans-2-Buten und Isobuten. 1-Buten und die beiden 2-Butene gehören dabei zur Gruppe der linearen Butene, während Isobuten ein verzweigtes Olefin darstellt. Die linearen $C_4$-Olefine 1-Buten, cis-2-Buten und trans-2-Buten werden in der Literatur oft als "n-Buten" zusammengefasst. Abhängig von den thermodynamischen Gegebenheiten treten die vier isomeren $C_4$-Olefine meist gemeinsam auf. Deswegen wird hier bei der Verwendung des Begriffs "Buten" zwischen Einzahl und Mehrzahl nicht unterschieden. Sofern hier ohne weitere Angaben von "Buten" die Rede ist, ist ein lineares Alken mit vier Kohlenstoffatomen (bzw. n-Buten) oder ein Gemisch enthaltend unterschiedliche isomere Alkene mit vier Kohlenstoffatomen gemeint.

Einen aktuellen Überblick über die chemischen und physikalischen Eigenschaften der Butene sowie deren technische Aufarbeitung und Verwertung bieten:

F. Geilen, G. Stochniol, S. Peitz and E. Schulte-Koerne: Butenes. Ullmann's Encyclopedia of Industrial Chemistry. (2013)

Butene fallen heute vorwiegend beim Cracken von Erdölfraktionen in einem Steamcracker oder in einem fluidkatalytischen Cracker (FCC) an und werden als Intermediat für die Herstellung vielfältiger Industrie-Chemikalien verwendet.

Unter einem "Hexen" ist im Folgenden ein Olefin mit sechs Kohlenstoffatomen zu verstehen oder ein Gemisch enthaltend mehrere unterschiedliche $C_6$-Olefine. Bei dem Begriff "Hexen" wird deswegen nicht zwischen Singular und Plural unterschieden. Zu den $C_6$- Olefinen gehören nämlich die achtzehn Isomeren 1-Hexen, (E)-2-Hexen, (Z)-2-Hexen, (E)-3-Hexen, (Z)-3-Hexen, 2-Methyl-1-penten, 2-Methyl-2-penten, (R)-3-Methyl-1-penten, (S)-3-Methyl-1-penten, (E)-3-Methyl-2-penten, (Z)-3-Methyl-2-penten, 4-Methyl-1-penten, (E)-4-Methyl-2-penten, (Z)-4-Methyl-2-penten, (3S)-2,3-Dimethyl-1-buten, (3R)-2,3-Dimethyl-1-buten, 2,3-Dimethyl-2-buten und 3,3-Dimethyl-1-buten. Von industriellem Interesse sind jedoch nur die Substanzen 1-Hexen und 4-Methyl-1-penten, die als Monomer oder Co-Monomer bei der Herstellung von Kunststoffen eingesetzt werden. Zu diesem Zwecke werden sie im Zuge der Oligomerisierung aus Ethen oder aus dem $C_3$-Olefin Propen hergestellt. Die Oligomerisierung wird später eingehend erläutert.

Unter Octen ist ein Olefin mit acht Kohlenstoffatomen zu verstehen oder ein Gemisch enthaltend mehrere unterschiedliche $C_8$-Olefine. Zu den $C_8$- Olefinen gehört eine Vielzahl von Isomeren, deren Aufzählung hier zu weit führt. Ein industriell wichtiger Vertreter der $C_8$-Olefine ist 1-Octen, welches durch Oligomerisierung von Ethen hergestellt wird und als Co-Monomer in Polyethylen eingesetzt wird.

Ein alternativer Weg zur Herstellung von Octen besteht in der Dimerisierung von n-Buten. Das dabei entstehende Gemisch aus Olefinen mit acht Kohlenstoffatomen wird als Dibuten bezeichnet, es ist also ein besonderes Octen im Sinne der hier gebrauchten Terminologie. Die Besonderheit des Dibutens liegt in der Isomerenverteilung, durch die es sich von anderen Octengemischen unterscheidet.

Je nachdem, auf welche Art und Weise sich die einzelnen n-Buten-Moleküle im Zuge der Oligomerisierung verbinden, erhält man ein Oligomerisat mit unterschiedlichem Verzweigungsgrad. Der Verzweigungsgrad wird durch den Isoindex beschrieben, welcher die mittlere Anzahl der Methylgruppen pro $C_8$-Molekül in dem Isomerengemisch angibt. Der Isoindex für Dibuten ist wie folgt definiert:

$$Isoindex = (Gewichtsanteil\ Methylheptene + 2* Gewichtsanteil\ Dimethylhexene) / 100$$

So tragen n-Octene mit 0, Methylheptene mit 1 und Dimethylhexene mit 2 zum Isoindex eines Produktgemisches aus $C_8$-Olefinen bei. Je niedriger der Isoindex, desto weniger verzweigt sind die Moleküle innerhalb des Gemisches aufgebaut. Das Gegenteil von verzweigt ist in diesem Zusammenhang linear. Ein Produkt mit einer hohen Linearität weist dementsprechend einen geringen Verzweigungsgrad auf.

Ein geringer Verzweigungsgrad ist immer dann bedeutsam, wenn das Olefingemisch als Ausgangsstoff für die Herstellung von Weichmachern eingesetzt werden soll. Wissenschaftliche Untersuchungen belegen, dass der Verzweigungsgrad von Olefingemischen, die durch Hydroformylierung, Hydrierung und Veresterung zu Weichmachern weiterverarbeitet werden, für die Eigenschaften und Qualität des Weichmachers maßgeblich ist.

Der Isoindex, den ein $C_8$-Olefingemisch erreichen muss, um als Ausgangsstoff für hochwertige Weichmacher dienen zu können, hängt von den jeweiligen Anforderungen der Weichmacherkunden ab und ändert sich mit der Zeit. Gegenwärtig wird meist ein Isoindex kleiner als 1.1 gefordert.

Unter der inzwischen bereits mehrfach erwähnten Oligomerisierung versteht man die Reaktion von Kohlenwasserstoffen mit sich selbst, wobei entsprechend längerkettige Kohlenwasserstoffe entstehen. Olefine mit zwei bis acht Kohlenstoffatomen lassen sich recht gut oligomerisieren.

So lässt sich beispielsweise durch die Oligomerisierung von zwei Olefinen mit drei Kohlenstoffatomen ein Olefin mit sechs Kohlenstoffatomen (Hexen) aufbauen. Die Oligomerisierung von zwei Molekülen miteinander wird auch Dimerisierung genannt. Verbinden sich hingegen drei Olefine mit drei Kohlenstoffatomen miteinander (Trimerisierung), entsteht ein Olefin mit neun Kohlenstoffatomen. Wird n-Buten einer Oligomerisierung unterworfen, entstehen dabei im Wesentlichen Olefine mit acht Kohlenstoffatomen (genauer gesagt: Dibuten) und dazu Olefine mit zwölf Kohlenstoffatomen ($C_{12}$-Olefine, "Tributen"), sowie in einem geringeren Umfang Olefine mit mehr als zwölf Kohlenstoffatomen ($C_{12}$+-Olefine, genannt "Tetrabuten").

Ein industriell praktiziertes Verfahren zur Herstellung von Dibuten durch Oligomerisierung von n-Buten ist der OCTOL®-Prozess. Die ausführliche Beschreibung desselben findet sich in der Nicht-Patentliteratur, beispielsweise unter:

B. Scholz: The HÜLS OCTOL Process: Heterogeneously catalyzed dimerization of n-butenes and other olefins. DGMK-Tagung in Karlsruhe, veröffentlicht in Erdöl, Erdgas, Kohle, April 1989, Seiten 21 und 22.
R.H. Friedlander, D.J. Ward, F. Obenaus, F. Nierlich, J. Neumeister: Make plasticizer olefins via n-butene dimerization. Hydrocarbon Processing, February 1986, Seiten 31 bis 33.
F. Nierlich: Oligomerize for better gasoline. Hydrocarbon Processing, February 1992, Seiten 45 bis 46.

Innerhalb der Patentliteratur beschreibt beispielsweise DE102008007081A1 eine auf dem OCTOL®-Prozess beruhende Oligomerisierung. EP1029839A1 befasst sich mit der Fraktionierung der in dem OCTOL®-Prozess entstehenden $C_8$-Olefine.

Der vollständig heterogen katalysierte OCTOL®-Prozess liefert ein Dibuten mit einem geringen Verzweigungsgrad, welches sich hervorragend zur Herstellung von Weichmachern eignet. Heterogen katalysiert bedeutet, dass der Katalysator als Feststoff in dem flüssigen bzw. gasförmigen Reaktionsgemisch vorliegt. Der Katalysator wird somit von den fluiden Reaktanden umspült und verbleibt im Reaktor. Da der OCTOL®-Prozess auf die Verarbeitung von $C_4$-Olefinen als Einsatzstoff optimiert ist, ist er von der Verfügbarkeit von Butenen als Einsatzstoff abhängig. Andere Rohstoffquellen lassen sich nicht ohne Mehraufwand verarbeiten. Da $C_6$-Olefine nicht durch Oligomerisierung von C4-Olefinen erhältlich sind, ist die Herstellung von Hexen mit dem OCTOL®-Prozess nicht möglich. Es werden ausschließlich Dibuten, Tributen und Tetrabuten gebildet. Der OCTOL®-Prozess ist insoweit unflexibel.

Eine größere Flexibilität hinsichtlich der Zielprodukte kann man durch Co-Oligomerisierung erreichen. Unter Co-Oligomerisierung versteht man die gleichzeitige Oligomerisierung von mehreren Substraten in einem Reaktionsgefäß. So beschreibt EP2582648B1 die Co-Oligomerisierung von Buten und Octen unter Erhalt von Dodecen ($C_{12}$-Olefin). Wie in jeder Oligomerisierung weiß man bei einer Co-Oligomerisierung nicht so genau, welches Olefin mit welchem reagiert: So kann in dem Beispiel der EP2582648B1 ein Dodecen sowohl aus drei Butenen, als auch aus einem Buten und einem Octen entstehen. Aus chemischer Sicht kann jede Oligomerisierung als eine Co-Oligomerisierung aufgefasst werden. Aus technischer Sicht hingegen liegt eine Co-Oligomerisierung jedoch nur dann vor, wenn mindestens zwei hinsichtlich ihrer Kohlenstoffanzahl unterschiedliche Olefine in einen gemeinsamen Reaktor eingebracht werden. Es kommt bei der Begriffswahl also auf die steuerbare Zuführung der Einsatzstoffe an, nicht auf die tatsächlich stattfindende Reaktion. Der in EP2582648B1 beschriebene Prozess ist hinsichtlich seiner Zielprodukte C8 und C12 schon flexibler als eine Oligomerisierung, die als Einsatzstoff ausschließlich C4 verwendet. Gleichwohl ist aber die Herstellung von Hexen ebenfalls nicht möglich. Dies geht nur durch Oligomerisierung von Ethen und/oder Propen.

WO2005/123884 offenbart die kombinierte Herstellung von 1-Octen und 1-Hexen durch Tetramerisierung und Trimerisierung von Ethylen. Dafür sind in einem gemeinsamen Reaktionsgefäß zwei unterschiedliche homogene Katalysatoren vorgesehen, nämlich ein erster für die Tetramerisierung und ein zweiter für die Trimerisierung. Da die eingesetzten Homogenkatalysatoren im Reaktionsgemisch gelöst sind, müssen sie daraus entweder abgetrennt werden oder darin verbleiben. Der letztere Fall ist dann kein Problem, wenn das Reaktionsgemisch als Co-Monomer in einer Polyethylen-Synthese eingesetzt wird. Bei der Herstellung von Polyethylen werden nämlich ausschließlich homogene Katalysatoren eingesetzt, die in dem Polymer verbleiben und damit verloren gehen. Wollte man das hergestellte 1-Hexen und 1-Octen aber gesondert abtrennen und möglichst als Reinsubstanzen erhalten, müssten die gelösten Homogenkatalysatoren erst aufwendig abgetrennt werden. Das Verfahren ist also zur isolierten Herstellung von 1-Octen und 1-Hexen kaum geeignet, es macht technisch nur im Verbund mit einer Polyethylen-Synthese Sinn.

Darüber hinaus erscheint dieses Verfahren auch nicht dazu geeignet, $C_8$-Olefine für den Einsatz als Ausgangsprodukt für Weichmacher herzustellen: Zwar fallen bei der kombinierten Tetra- und Trimerisierung auch bis zu 52 Gew-% $C_8$-Olefine an, jedoch ohne genaue Angaben zu dem Verzweigungsgrad. Das Verfahren ist im Übrigen auch auf die Produktion des Co-Monomers 1-Octen optimiert, ein $C_8$-Olefin, was sich ohnehin kaum zur Weichmacherherstellung eignet. Es ist daher nicht ersichtlich, dass die $C_8$-Alkene einen Iso-Index erreichen, der sie als Ausgangsprodukt zur Weichmacherherstellung qualifiziert. Darüber hinaus wäre der homogen gelöste Katalysator bei dieser Verwendung definitiv abzutrennen, da die anschließende Hydroformylierung ebenfalls homogen katalysiert wird und auf Störungen von eingeschleppten Fremd-Katalysatoren empfindlich reagiert.

Das eben Gesagte gilt auch für das in WO2005/123633 offenbarte Verfahren zur Oligomerisierung von Ethylen, welches in Gegenwart von Cyclohexan durchgeführt wird. Das Cyclohexan dient dabei als Lösemittel und soll die Desaktivierung des eingesetzten Homogenkatalystors bzw. seines Aktivators verringern.

Ähnliches gilt auch für US2013/0066128 A1 betreffend die homogene Oligomerisierung von Ethen in n-Heptan.

Das Problem der Katalysatorabtrennung besteht nicht bei heterogen katalysierten Prozessen, bei denen der Katalysator als Feststoff vorliegt und im Reaktor verbleibt. Ethylen-Oligomerisierung an einem festen Si/Al/Ni-System ist in US8637722B2 beschrieben. Dieser Prozess findet allerdings in der Gasphase statt, was nachteilig bei der Raumausnutzung der Reaktoren ist. Zudem finden die etablierten Prozessschritte der Weiterverarbeitung von Butenen und Octenen in der Flüssigphase statt, sodass dieser Gasphasenprozess mit bestehender Technologie nicht ohne weiteres kompatibel ist. Eine notwenige Verflüssigung der in der Gasphase gewonnenen Butene und Octene erfordert zusätzliche Energie.

Auch der in WO2010/117539A1 gezeigte Gasphasenprozess zur Oligomerisierung von in einem FCC-Gas verdünnten Ethylen an einem zeolithischen Ni-Katalysator lässt sich nicht ohne weiteres in einen etablierten Produktionsstrang zur $C_4/C_8$ Verwertung eingliedern.

Dasselbe gilt auch für die in US4717782 beschriebene, heterogene Gasphasen-Oligomerisierung von Ethen an einem Nickel-haltigen Zeolith. Das Einsatzgemisch kann auch $C_4$-Paraffine und inerte Gase enthalten. Auch US8637722 beschreibt die Oligomerisierung von Ethen in der Gasphase an einem heterogenen Katalysator aus Ni/Al auf einem Träger aus $Al_2O_3SiO_2$. Inerte Gase wie Stickstoff, Argon oder Helium können zugegen sein.

Eine Mischform zwischen heterogener und homogener $C_2$-Oligomerisierung zeigt US2013/0158321A1. Hier wird Ethen zunächst homogen zu Butenen dimerisiert und diese anschließend durch heterogene Katalyse an einem festen Nickelkontakt in Octene umgesetzt. Beide Reaktionsstufen finden in der flüssigen Phase in Gegenwart von Hexan statt. Der Reaktionsaustrag der ersten Stufe muss mit Base neutralisiert und destillativ von dem Homogenkatalysator (Triethylaluminium) befreit werden. In der industriellen Praxis ist dies sehr aufwendig.

US2581228 beschreibt die heterogen katalysierte Oligomerisierung von Ethen in Gegenwart eines inerten Lösemittels. Bei dem Lösemittel soll es sich um ein höher siedendes, inertes Material handeln, vorzugsweise ein höher siedendes Alken oder Cycloalken. Als Katalysator wird ein Nickel/ Aluminium-System auf Silica Gel eingesetzt. Das Reaktionsgemisch ist ein Slurry, aus dem der gelförmige Katalysator zurückgewonnen werden kann. Dafür ist entsprechender apparativer Aufwand zu leisten.

Im Lichte dieses Standes der Technik liegt der vorliegenden Erfindung die Aufgabe zu Grunde, ein vollständig heterogen katalysiertes Verfahren zur kombinierten Herstellung von zumindest Buten und Octen aus Ethen anzugeben, der ohne eine aufwändige Katalysatorabtrennung betrieben werden kann und dennoch nachgelagerte, homogen katalysierte Prozesse nicht verunreinigt. Die erhaltenen Produkte sollen möglichst linear sein. Außerdem soll der Prozess möglichst in der flüssigen Phase durchgeführt werden können, um mit etablierten Technologien zur Verwertung von Buten und Octen kompatibel zu sein. Dies spart kostspielige Neuentwicklungen innerhalb eines komplexen Produktionsverbundes. Darüber hinaus soll der Prozess in der Lage sein, neben den beiden Zielprodukten Buten und Octen zusätzlich auch Hexen als drittes Zielprodukt herzustellen. Die Nachfrage nach diesen Olefinen kann nämlich schwanken. Der Prozess soll dementsprechend auf sich ändernde Marktbedürfnisse reagieren können. Dies ist eine weitere wesentliche Aufgabe, die der Erfindung zu Grunde liegt.

Gelöst werden diese Aufgaben durch ein Verfahren mit den folgenden Schritten:

a) Bereitstellen eines inerten Lösemittels, dessen Siedepunkt bzw. Siedebereich unterhalb der Siedepunkte der Butene liegt;

b) Bereitstellen eines ersten Einsatzgemisches enthaltend zumindest das inerte Lösemittel und darin gelöstes Ethen;

c) Überführen des ersten Einsatzgemisches in eine erste Synthese;

d) in der ersten Synthese Oligomerisieren zumindest eines Teils des im ersten Einsatzgemisch enthaltenden Ethens in Gegenwart eines ersten heterogenen Katalysators und in Gegenwart des inerten Lösemittels unter Erhalt eines ersten Reaktionsgemisches umfassend zumindest das inerte Lösemittel, Buten, Hexen und Octen;

e) Aufarbeiten des ersten Reaktionsgemisches und/oder eines darauf basierenden Stoffstroms in eine Leichtsiederfraktion enthaltend das inerte Lösemittel, mindestens eine $C_4$-Fraktion enthaltend die Butene, eine $C_6$-Fraktion enthaltend die Hexene, eine $C_8$-Fraktion enthaltend die Octene und in eine $C_{8+}$-Fraktion enthaltend Kohlenwasserstoffe mit mehr als acht Kohlenstoffatomen;

f) Verwenden zumindest eines Teils der Leichtsiederfraktion beim Bereitstellen des ersten Einsatzgemisches;

g) Bereitstellen eines zweiten Einsatzgemisches enthaltend zumindest Hexen sowie im Hexen gelöstes Ethen unter Verwendung zumindest eines Teils der $C_6$-Fraktion;

h) Überführen des zweiten Einsatzgemisches in eine zweite Synthese, wobei die zweite Synthese von der ersten Synthese räumlich getrennt ist;

i) in der zweiten Synthese Umsetzen zumindest eines Teils des im zweiten Einsatzgemisch enthaltenden Ethens mit zumindest einem Teil des im zweiten Einsatzgemisch enthaltenden Hexens in Gegenwart eines zweiten heterogenen Katalysators unter Erhalt eines zweiten Reaktionsgemisches umfassend zumindest Octen;

k) Gemeinsame Aufarbeitung des zweiten Reaktionsgemisches mit dem ersten Reaktionsgemisch bzw. mit dem darauf basierenden Stoffstrom.

Ein solches Verfahren ist Gegenstand der Erfindung.

Das Grundkonzept des vorliegenden Verfahrens besteht darin, Buten, Hexen und Octen gleichzeitig durch Oligomerisierung von Ethylen in einem inerten Lösungsmittel an einem heterogenen Katalysator herzustellen. Die Verwendung des heterogenen Katalysators hat zunächst den Vorteil, dass die Abscheidung aus dem Reaktionsgemisch einfacher ist als bei einem homogen katalysierten Prozess. Einfachstenfalls wird ein Festkörper als heterogener Katalysator eingesetzt, der in dem Reaktor verbleibt, während das fluide Reaktionsgemisch aus dem Reaktor abgezogen wird. Eine gesonderte Katalysatorabtrennung entfällt dann praktisch.

Ein wesentlicher Aspekt der Erfindung besteht darin, dass die Reaktion in Gegenwart eines inerten Lösemittels durchgeführt wird. Genauer gesagt wird das Ethylen in dem Lösemittel gelöst, die Reaktion findet in dem Lösemittel statt und das erhaltene Reaktionsgemisch wird mit Hilfe des Lösemittels aus dem Reaktor herausgefördert. Das Lösemittel dient mithin als Transportmedium für die Edukte in den Reaktor und der Produkte aus dem Reaktor wieder raus. Dies erleichtert im industriellen Maßstab die Handhabung der Edukte und Produkte und ermöglicht erst die einfache Katalysatorabtrennung, die einfachstenfalls eine Abtrennung einer Flüssigkeit (Reaktionsgemisch) von einem Festkörper (Katalysator) darstellt. Ein weiterer Vorteil der Durchführung der Oligomerisierung in einem inerten Lösemittel ist, dass die bei der Oligomerisierung anfallende Reaktionswärme aus dem Reaktor abtransportiert werden kann. Damit lässt sich die Reaktion besser beherrschen und auch die Produktbildung beeinflussen, da die unterschiedlichen Oligomere unterschiedlich schnell und bei unterschiedlichen Temperaturen gebildet werden. Über das Lösemittel wird also nicht nur die Verweilzeit, sondern auch die Temperatur der ersten Synthese gesteuert.

Die Wahl des richtigen Lösemittels ist nicht beliebig; vielmehr muss es zwei wichtige Voraussetzungen erfüllen: Zum einen muss es sich in der ersten Synthese, also der Ethen-Oligomerisierung, inert verhalten. Dies bedeutet, dass es in der ersten Reaktion nicht verbraucht werden darf und somit weiter als Transportmedium für die Produkte und die Reaktionswärme zur Verfügung steht. Die zweite wesentliche Eigenschaft des inerten Lösemittels besteht in seiner Siedepunktlage im Verhältnis zu den in der ersten Synthese gebildeten Butenen: Das erfindungsgemäß eingesetzt Lösemittel muss zwingend einen Siedepunkt haben, der unterhalb des Siedepunkts der Butene liegt, denn nur so ist es möglich, das Lösemittel mitsamt des unverbrauchten Ethens aus dem Reaktionsgemisch über Kopf abzudestillieren und zur Bereitstellung des ersten Einsatzgemisches wiederzuverwerten (zu rezyklieren). Je größer der Abstand der Siedepunkte des Lösemittels und der Butene ist, desto einfacher und energiesparender lässt sich das Lösemittel abtrennen, da es ja zur Abtrennung verdampft werden muss. Die genaue Siedepunktlage des Lösemittels hängt davon ab, welche Butene in der ersten Synthese gebildet werden. Der Siedepunkt des Lösemittels sollte unterhalb des Siedepunkts des Butens im System liegen, das den niedrigsten Siedepunkt hat. Ein Siedepunkt unter dem von Isobuten (-6.9°C) sollte ausreichen, falls Isobuten aber in der Oligomerisierung nicht gebildet wird, genügt ein Siedepunkt unterhalb dem von 1-Buten (-6.3°C). Im Regelfall wird bei der Oligomerisierung von Ethen kein Isobuten gebildet, sodass ein Siedepunkt unter -6.3°C ausreicht. Die Siedepunkte werden bei denselben Druckverhältnissen verglichen. Die eben genannten Temperaturangaben verstehen sich bei Normaldruck (1013 hPa). Sofern das inerte Lösemittel keinen singulären Siedepunkt hat, sondern einen Siebereich - etwa weil es sich bei dem Lösemittel nicht um einen Reinstoff, sondern um ein Gemisch handelt, dessen Bestandteile unterschiedliche Siedepunkte aufweisen - dann muss die obere Grenze des Siedebereichs des Lösemittels unterhalb des niedrigsten Siedepunkts der gegenwärtigen Butene liegen. Der Grund, warum das inerte Lösemittel erfindungsgemäß eine niedrigere Siedepunktlage haben muss als die Butene, besteht darin, dass das inerte Lösemittel sich so frühzeitig aus dem Aufarbeitungsstrang abscheiden und zurückführen lässt, weswegen es stromabwärts gelegene Aufarbeitungsstufen nicht hydraulisch belastet.

Die frühzeitige Abtrennbarkeit des Lösemittels ist auch deshalb bedeutsam, da erfindungsgemäß neben der Ethen-Oligomerisierung noch eine zweite Synthese vorgesehen ist, die ebenfalls nicht mit inertem Lösemittel beaufschlagt wird.

Ein weiterer wesentlicher Gesichtspunkt der Erfindung besteht nämlich darin, dass die in der ersten Reaktion (Ethen-Oligomerisierung) gebildeten Hexene in einer zweiten Reaktion mit weiterem Ethylen zu Octenen umgesetzt werden. Grund dafür ist, dass in der ersten Synthese die $C_4$-, die $C_6$- und die $C_8$-Oligomere mit abfallender Selektivität gebildet werden, also am meisten Butene, dann Hexene und dann die Octene. Um insgesamt eine größere Ausbeute an Octenen zu erreichen, werden die Hexene mit Ethen in einer zweiten, separaten Synthese weiter zu Octenen umgesetzt. Dabei dient das Hexen als Lösemittel für das Ethen. Es verhält sich aber in der zweiten Reaktion definitiv nicht inert, sondern wird gezielt umgesetzt und zwar möglichst vollständig. Gleichwohl übernimmt das Hexen in der zweiten Reaktion eine Funktion wie das inerte Lösemittel in der ersten Reaktion, nämlich den Transport von Ethen in den Reaktor. Der Transport des Reaktionsproduktes und der Reaktionswärme aus dem zweiten Reaktor wieder heraus erfolgt aber mit dem zweiten fluiden Reaktionsgemisch. Dabei wird wieder der Vorteil der einfachen Katalysatorabtrennung erzielt.

Je nachdem, ob mit dem Prozess tendenziell mehr Hexene oder mehr Octene produziert werden sollen, werden die in der ersten Synthese gebildeten Hexene entweder komplett in die zweite Synthese gefahren oder nur ein Teil der $C_6$-Fraktion. Die übrigen Hexene können dann neben dem Buten und dem Octen als drittes Zielprodukt veräußert werden. Es ist aber auch möglich, lediglich Buten und Octen als Zielprodukt auszuschleusen und das zwangsweise in der ersten Synthese gebildete Hexen in der zweiten Synthese komplett zu Octen umzusetzen, sodass das Hexen gar nicht den Prozess verlässt. Die Hexen-Bilanz über den Gesamtprozess kann also wahlweise neutral oder positiv sein. Dies ist nur deshalb möglich, weil die beiden Reaktionen räumlich voneinander getrennt durchgeführt werden und weil sich die Umsätze und die Selektivitäten in den beiden separaten Reaktionen Dank der Verwendung des inerten Lösemittels in der ersten Reaktion bzw. des Hexens als nicht inertes Lösemittel in der zweiten Reaktion aufeinander anpassen lassen.

Ein weiterer wesentlicher Aspekt der Erfindung ist, dass die Reaktionsausträge beider Synthesen gemeinsam aufgearbeitet werden. Mithin ist nur eine Sequenz aus Destillationskolonnen vorgesehen, die mit beiden Reaktionsgemischen beaufschlagt wird und so die in den von der Kolonnensequenz abgetrennten Fraktionen enthaltenden Olefine aus beiden Reaktionen stammen können. Der Mehraufwand der doppelten Reaktionsführung bedeutet somit keinen destillativen Mehraufwand.

Konkret wird der erfindungsgemäße Prozess so geführt, dass mit Hilfe einer ersten Destillationskolonne die Leichtsiederfraktion über Kopf aus dem ersten Reaktionsgemisch bzw. aus dem auf dem ersten Reaktionsgemisch basierenden Stoffstrom abgetrennt wird unter Erhalt eines im Wesentlichen lösemittelfreien Sumpfprodukts, aus welchem wiederum die $C_4$-Fraktion, die $C_6$-Fraktion, die $C_8$-Fraktion und die $C_{8+}$-Fraktion abgetrennt werden. Das Merkmal "Lösungsmittelfrei" bezieht sich auf das in der ersten Synthese eingesetzte inerte Lösemittel (das Sumpfprodukt der ersten Destillation enthält erfindungsgemäß durchaus ein Lösemittel, nämlich Hexen, welches als Lösemittel für die zweite Synthese verwendet wird; allerdings ist dies nicht inert, sondern reaktiv). Sofern das inerte Lösemittel einen deutlichen Siedepunktabstand von den produzierten Butenen hat, ist es vertretbar, das inerte Lösemittel komplett abzutrennen, sodass das Sumpfprodukt vollständig frei von inertem Lösemittel ist. Wenn der Siedepunkt des Lösemittels jedoch sehr eng an dem der Butene liegt, bedeutet die vollständige Abtrennung des Lösemittels jedoch einen großen (energetischen) Aufwand. In solchen Konstellationen ist es vertretbar, geringe Mengen an inertem Lösemittel im Sumpf der ersten Destillation zu lassen. Dieser wäre dann auch "im Wesentlichen lösemittelfrei" im Sinn der Erfindung. Vorzugsweise enthält das im Wesentlichen lösemittelfreie Sumpfprodukt weniger als 10 Gew.-% inertes Lösemittel, besser noch weniger als 1 Gew.-% und optimal weniger als 0.2 Gew.-% inertes Lösemittel.

Die Leichtsiederfraktion, die hinter der ersten Synthese abgetrennt und davor zurückgeführt wird, enthält in machen Varianten der Erfindung neben dem inerten Lösemittel noch Ethen, welches in der ersten Synthese und/oder in der zweiten Synthese nicht umgesetzt wurde. Dies ist physikalisch möglich, wenn die Siedepunktlage des Lösemittels zwischen Ethen und Buten liegt. Ethen liegt an dieser Stelle vor, wenn es in der ersten Synthese nicht vollständig umgesetzt wurde (etwa, weil die Verweildauer zu kurz war) oder wenn es in der zweiten Synthese nicht vollständig umgesetzt wurde (Ethen-Überschuss) und der zweite Reaktionsaustrag vor der ersten Destillationskolonne mit dem ersten Reaktionsgemisch verschnitten wird. Die Situation, dass in der ersten und/oder zweiten Synthese das Ethen nicht vollständig umgesetzt wird, kann insbesondere dann vorkommen, wenn der Prozess eingefahren wird, etwa nachdem auf eine veränderte Nachfrage reagiert wurde. Da die Ethylen-Rückführung über die Leichtsiederfraktion aber automatisch geschieht - die korrekte Siedepunktlage des inerten Lösemittels vorausgesetzt - brauchen keine apparativen Prozessänderungen vorgenommen zu werden; es genügt, den Zulauf von Frisch-Ethen beim Bereitstellen des ersten Einsatzgemisches zu reduzieren.

Mehrfach wurde bereits auf die Bedeutung der Siedepunktlage des inerten Lösemittels eingegangen. Beispiele für Reinsubstanzen, die alle hier geforderten Eigenschaften des inerten Lösemittels aufweisen, sind Propan und iso-Butan. Der Siedepunkt dieser $C_3$ bzw. $C_4$-Alkane liegt zwischen Ethen und Buten und ist daher zur Überkopf-Abtrennung geeignet. Methan und Ethan lassen sich indes nicht verwenden, da sie unter Reaktionsbedingungen gasförmig sind. Die Alkane verhalten sich in der Oligomerisierung verglichen mit Ethen und den anderen Olefinen inert. Anstelle der Reinsubstanzen kann auch ein Gemisch aus Propan und iso-Butan verwendet werden, was dann zu einem Siedebereich statt zu einem singulären Siedepunkt führt. In allen Mischungsverhältnissen führen diese beiden Alkane aber zu einem inerten Lösemittel mit geeigneter Siedepunktlage.

Die Reaktionsbedingungen in der ersten Synthese sollten wie folgt gewählt sein:

Temperatur: 20°C bis 150°C; Druck: $1*10^5$ Pa bis $50*10^5$ Pa; Raum/Zeit-Belastung: 3 bis 50 $h^{-1}$. Das molare Verhältnis von Ethen zu Lösemittel in dem ersten Einsatzgemisch sollte eingestellt sein auf einen Wert zwischen 0.1 und 0.5, wobei der Anteil des Ethens an dem ersten Einsatzgemisch und die Reaktionsbedingungen der ersten Synthese so gewählt sind, dass das Lösemittel in einer flüssigen Phase vorliegt. Durch die Wahl der Reaktionsbedingungen in den angegebenen Bereichen unter der Maßgabe, dass das Lösemittel in der ersten Reaktion flüssig ist, führt zu der einfachen Katalysatorabtrennung, zum effektiven Abtransport der Reaktionswärme und insgesamt zu einer hohen Prozessintensität.

Die Reaktionsbedingungen und der Anteil des Ethens an dem ersten Einsatzgemisch können in den angegeben Bereichen entweder so gewählt werden, dass das Ethen vollständig in dem flüssigen Lösemittel gelöst ist oder so, dass das Ethen lediglich teilweise in dem flüssigen Lösemittel gelöst ist und teilweise in einer Gasphase vorliegt. Wenn das Ethen vollständig in dem flüssigen inerten Lösemittel gelöst ist, findet die erste Synthese (Oligomerisierung) komplett in der flüssigen Phase statt. Dies ist im Interesse der Prozessintensität anzustreben.

Alternativ kann nicht gelöstes Ethen Gasblasen in dem flüssigen Einsatz- bzw. Reaktionsgemisch bilden. Die erste Synthese würde dann in der Sprudelphase durchgeführt. Dies hätte den Vorteil, dass gerade zu Anfang der Reaktion ein sehr hoher Anteil des Ethens verbraucht wird, so dass das in feinen Gasblasen ungelöst vorliegende überschüssige Ethen sich im folgenden Reaktorabschnitt wiederum ins Lösemittel nachlöst und es somit wieder anreichert. Zudem hat die faktisch daraus resultierende längere Präsenz des Ethens einen positiven Einfluss auf die $C_4$-gegenüber den $C_{4+}$-Selektivitäten und den Linearitäten der Alkene: da Verzweigungen durch Desorption und Re-Adsorption an das aktive Katalysatorzentrum zustande kommen und Ethen bei der Re-Adsorption erfolgreich um die Bindungsstellen konkurriert, kommt es zu kürzeren Ketten und zu einer Bevorzugung des alternativen Mechanismus (fortschreitende Kettenverlängerung statt Desorption/Adsorption). Insgesamt bedeutet dies, dass in der Sprudelphase verstärkt lineare 1- und 2-Alkene gebildet werden.

Im Interesse der Prozessintensität sollten bei der zweiten Synthese (Umsetzung Ethen mit Hexen zu Octen) die Reaktionsbedingungen so gewählt werden, dass der Anteil des Ethens an dem zweiten Einsatzgemisch zwischen 0.1 Gew.-% und 30 Gew.-%, beträgt, und dass die zweite Synthese bei einer Temperatur zwischen 20°C und 150 °C und bei einem Druck zwischen $1*10^5$ Pa und $50*10^5$ Pa erfolgt, wobei der Anteil des Ethens an dem zweiten Einsatzgemisch und die Reaktionsbedingungen der zweiten Synthese so gewählt sind, dass das Hexen in einer flüssigen Phase vorliegt und das Ethen darin vollständig gelöst ist. Eine Sprudelphase in der zweiten Reaktion ist nicht gewollt, da dadurch mehr $C_4$ entstehen würde. Anteil des Ethens im zweiten Einsatzgemisch sollte möglichst klein gewählt werden, damit das Ethen möglichst vollständig umgesetzt wird. Vorzugsweise weist das zweite Einsatzgemisch weniger Ethen als Hexen auf, der Ethen-Anteil im zweiten Einsatzgemisch sollte kleiner sein als 30 Gew.-% besser noch geringer als 20 Gew.-%.

Optimal wird der Prozess so gefahren, dass das in der zweiten Synthese benötigte Hexen vollständig in der ersten Synthese gebildet wird. Das erste Reaktionsgemisch wird mithin ausschließlich unter Verwendung zumindest eines Teils der $C_6$-Fraktion und unter Zugabe von Ethen bereitgestellt. Dies bedeutet, dass von außen kein Hexen zugeführt werden muss. Die Hexen-Bilanz über den gesamt-Prozess ist dann nicht negativ. Nicht negativ bedeutet entweder neutral oder positiv.

Im Fall einer neutralen Hexen-Bilanz wird das zweite Reaktionsgemisch ausschließlich unter Verwendung der gesamten $C_6$-Fraktion und unter Zugabe von Ethen bereitgestellt. Dies bedeutet, dass das komplette Hexen, welches in der ersten Synthese gebildet wird, in der zweiten Synthese zu Octen wieder verbraucht wird. Über den gesamten Prozess bilanziert wird also gar kein Hexen produziert sodass Buten und Octen die einzigen Zielprodukte sind, die ausgeschleust werden.

Im Falle einer positiven Hexen-Bilanz wird das zweite Reaktionsgemisch ausschließlich unter Verwendung eines ersten Teils der $C_6$-Fraktion und unter Zugabe von Ethen bereitgestellt und ein zweiter Teil der C6-Fraktion wird ausgeschleust. In diesem Fall ist Hexen neben Buten und Octen ein drittes Zielprodukt. Dies setzt allerdings eine Überproduktion von Hexen in der ersten Synthese gegenüber dem Verbrauch in der zweiten Synthese voraus. Die Produktion von Octen nimmt damit zu Gunsten der Produktion von Hexen ab.

Der Prozess kann auch zwischen einer neutralen und positiven Hexen-Bilanz wechselnd gefahren werden, wenn sich die Nachfrage nach Hexen bzw. Octen gegenläufig verändert. Apparativ muss an der Anlage nichts geändert werden, lediglich die Reaktionsbedingungen sollten verändert werden, um die in der ersten und zweiten Synthese eingesetzten Katalysatoren zu unterschiedlicher Selektivität zu veranlassen.

Ein heterogener Katalysator, der dies beherrscht, enthält mindestens zwei Komponenten, wobei die erste Komponente mindestens ein aus Ni, Cr, Fe, Ti ausgewähltes Element umfasst, welches in metallischer und/oder oxidischer und/oder hydridischer Form vorliegt, und wobei die zweite Komponente mindestens ein aus $Al_2O_3$, $SiO_2$, $TiO_2$, $ZrO_2$ ausgewähltes Metalloxid umfasst. Ein Beispiel eines solchen Katalysators ist aus US2581228 bekannt.

Ein besonderer Vorteil eines hier charakterisierten Katalysators ist, dass er sich sowohl in der ersten Synthese als auch in der zweiten Synthese einsetzen lässt, da er beide Reaktionen katalysiert. Seine Selektivität in der jeweiligen Reaktion lässt sich durch Veränderung der Reaktionsbedingungen beeinflussen, sodass mit ihm abhängig von der Bedarfslage mehr Buten, mehr Hexen oder mehr Octen produziert werden kann. In der Oligomerisierung in inerten Lösemitteln bildet er aber stets mehr $C_4$-Oligomere als $C_6$-Oligomere und die mehr als $C_8$-Oligomere und höherer Oligomere ($C_{8+}$). In Hexen als Lösungsmittel verschiebt sich jedoch das Verhältnis zu mehr $C_8$ und weniger $C_6$, sodass mehr Octen als Hexen gebildet wird. Überraschend ist, dass dieser Katalysator auch die Umsetzung von $C_2$ und $C_6$ nach $C_8$ beschleunigt und deswegen auch in der zweiten Synthese einsetzbar ist. Dies ist aus der US2581228 nicht ableitbar. Es kann also derselbe Katalysator in beiden Reaktionen eingesetzt werden. Es ist aber auch möglich, zwei unterschiedliche Katalysatoren zu nutzen, die auf die jeweilige Reaktion optimiert sind.

Anhand der beiliegenden Figuren sollen nun einige Beispiele für einen erfindungsgemäßen Prozess näher erläutert werden. Es zeigen:

Figur 1: Fließbild Basisprozess
Figur 2: wie Figur 1, zusätzlich mit Isomerisierung
Figur 3: wie Figur 1, zusätzlich mit oxidativen Dehydrierung
Figur 4: wie Figur 1, zusätzlich mit nachgeschalteten OCTOL®-Prozess

Alle Figuren sind schematisch und zeigen lediglich die wesentlichen Bestandteile einer entsprechenden Anlage zur Durchführung des erfindungsgemäßen Verfahrens.

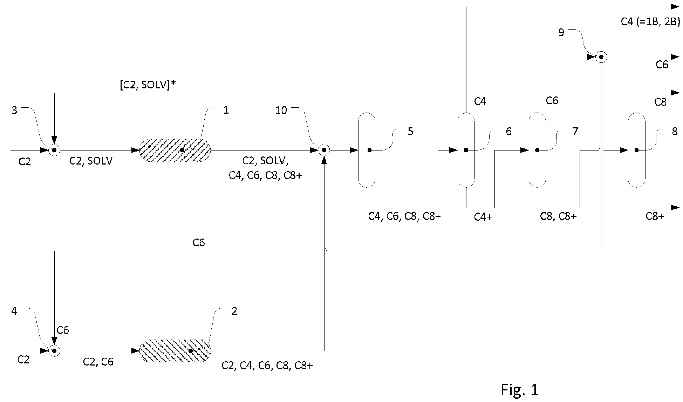

Figur 1 zeigt das Grundprinzip. Es sieht zwei parallel betriebene Synthesen 1, 2 vor, die in räumlich getrennten Reaktoren durchgeführt werden. Bei der ersten Synthese 1 handelt es sich um eine Ethen-Oligomerisierung. Sie dient vornehmlich zur Herstellung von Buten, produziert daneben aber auch Hexen und Octen. Die zweite Synthese 2 dient zur Herstellung von Octen aus Ethen und Hexen.

Das für beide Synthesen 1,2 benötigte Ethen C2 stammt aus einer oder mehreren hier nicht dargestellten Quellen. Die Reinheit des flüssig oder gasförmig anströmenden Ethens C2 beträgt mehr als 99,9%. Als Begleitstoffe können weniger als 10 ppm Sauerstoff, weniger als 5 ppm Kohlenmonoxid, weniger als 10 ppm Kohlendioxid und weniger als 1000 ppm anderer Kohlenwasserstoffe auftreten. Eine höhere Reinheit ist nicht erforderlich, da die häufigsten Verunreinigungen inerte Alkane wie Ethan oder Methan sind, welche die Reaktion selbst nicht stören und lediglich bei höheren Anteilen die Siede- und Druckbereiche leicht verändern.

Jeder Synthese 1,2 ist jeweils ein Mischer 3, 4 zugeordnet. Der erste Mischer 3 dient dazu, für die erste Synthese 1 ein erstes Einsatzgemisch C2, SOLV bereitzustellen. Bei dem ersten Einsatzgemisch handelt es sich um ein flüssiges Gemisch aus einem inerten Lösemittel SOLV und darin vollständig gelöstem Ethen C2. Als inertes Lösemittel SOLV kann Propan oder iso-Butan oder eine Mischung draus verwendet werden. Da sich das Lösemittel SOLV inert verhält, wird es im Prozess nicht verbraucht. Es kann daher im Kreislauf geführt werden. Deswegen stammt das Lösemittel SOLV, welches im ersten Mischer 3 zur Bereitstellung des ersten Einsatzgemisches C2, SOLV benötigt wird, aus einer zurückgeführten Leichtsiederfraktion [C2, SOLV]* deren Ursprung noch erläutert werden wird.

Die Zusammensetzung des ersten Einsatzgemisches wird in dem ersten Mischer 3 so eingestellt, dass es bei den Reaktionsbedingungen in der ersten Synthese flüssig ist und das Ethen vollständig in dem Lösemittel gelöst ist.

In der ersten Synthese 1 wird das Ethen in Gegenwart eines ersten heterogenen Katalysators und in Gegenwart des inerten Lösemittels SOLV oligomerisiert. Dabei entstehen als Dimere Butene C4, als Trimere Hexene C6, Octene C8 als Tetramere des Ethens sowie höhere Olefine C8+. Die Selektivität der ersten Synthese nimmt mit der Kettenlänge der Oligomere ab, es werden also vornehmlich Butene gebildet, danach Hexen und eher wenig Octen und kaum höhere Olefine C8+. Ein Teil des Ethens C2 wird nicht umgesetzt. Insgesamt umfasst der erste Reaktionsaustrag C2, SOLV, C4, C6, C8, C8+, also nicht umgesetztes Ethen sowie dessen Oligomere Buten, Hexen, Octen und höhere Olefine. Da sich das Lösemittel SOLV in der Reaktion inert verhält, findet es sich im ersten Reaktionsgemisch wieder.

Das erste Reaktionsgemisch C2, SOLV, C4, C6, C8, C8+, SOLV wird mit Hilfe einer Serie aus vier Kolonnen 5, 6, 7, 8 destillativ aufgearbeitet. Die erste Destillationskolonne 5 trennt über Kopf die bereits erwähnte Leichtsiederfraktion [C2, SOLV]* ab. Die Leichtsiederfraktion enthält nicht umgesetztes Ethen C2 sowie das Lösemittel SOLV. Da das Lösemittel aufgrund seiner Siedepunktlage unterhalb der Butene C4 siedet, bleiben die Oligomere C4, C6, C8 und C8+ im Sumpf der ersten Destillationskolonne 5. Der Sumpf der ersten Destillationskolonne 5 ist frei von dem inerten Lösemittel SOLV. Die nachfolgenden Prozessschritte werden mithin von dem Lösemittel nicht belastet. Stattdessen wird an der ersten Destillationskolonne das Lösemittel komplett wiedergewonnen und zum ersten Mischer 3 zurückgeführt. Dort wird es bei der Bereitstellung des ersten Einsatzgemisches C2, SOLV wiederverwertet. Bei dem angestrebten hohen Umsatz in der ersten Synthese sollte allerdings die Leichtsiederfraktion kaum Ethen enthalten.

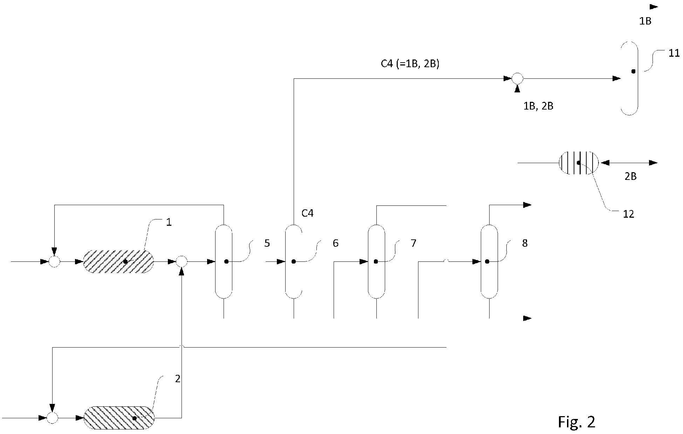

Die zweite Destillationskolonne 6 trennt nun über Kopf die aus dem ersten Reaktionsgemisch stammenden Butene C4 als eine $C_4$-Fraktion ab. Die $C_4$-Fraktion enthält im Wesentlichen 1-Buten 1B und cis/trans-2-Buten 2B. Die Butene sind das erste Zielprodukt des Prozesses. Sofern besonders Interesse an 1-Buten besteht, kann die C4-Fraktion noch weiter aufgearbeitet werden. Dies wird anhand der Figuren 2 bis 4 beschrieben.

Die Olefine mit mehr als vier Kohlenstoffatomen C4+ werden vom Sumpf der zweiten Destillationskolonne 6 in die dritte Destillationskolonne 7 geführt. Dort wird über Kopf eine C6-Fraktion abgetrennt, enthaltend die Hexene C6. Octen C8 und die höheren Olefine C8+ verbleiben im Sumpf der dritten Destillationskolonne 7.

Abhängig von der Nachfrage von Hexen als separates Zielprodukt wird die C6-Fraktion von Kopf der dritten Destillationskolonne an einem Teiler 9 geteilt in einen Teil, welcher aus dem Prozess ausgeschleust wird und in einen Teil, der zu dem zweiten Mischer 4 geführt wird. Falls Hexen nicht als drittes Zielprodukt separat nachgefragt wird, wird die komplette C6-Fraktion zu dem zweiten Mischer 4 geführt. In den Figuren 2, 3 und 4 ist ein solches Szenario dargestellt. Es ist aber nicht möglich, die komplette C6-Fraktion aus dem Prozess herauszuziehen, da das darin enthaltende Hexen in der zweiten Synthese als Lösemittel benötigt wird. In dem Teiler 9 findet keine Trennung der Fraktion statt, beide Teile haben also dieselbe Zusammensetzung und bestehen im Wesentlichen aus Hexen C6.

Im zweiten Mischer 4 wird in dem Hexen C6 aus der C6-Fraktion frisches Ethen C2 gelöst, sodass ein zweites Einsatzgemisch C2, C6 entsteht. Die Zusammensetzung des zweiten Einsatzgemisches C2, C6 wird in dem zweiten Mischer 4 so eingestellt, dass es bei den Reaktionsbedingungen in der zweiten Synthese 2 flüssig ist und das Ethen vollständig im Hexen gelöst ist.

In der zweiten Synthese 2 wird nun heterogen katalysiert Ethen C2 und Hexen C6 zu Octen C8 umgesetzt. Daneben finden in der zweiten Synthese 2 noch Nebenreaktionen statt, denn es werden dort auch Butene C4 und höhere Olefine C8+ gebildet. Im Übrigen ist es auch vorstellbar, dass in der zweiten Synthese Octen durch Tetramerisierung von Ethen entsteht.

Falls an Hexen als separates Verkaufsprodukt kein Interesse besteht, wird die zweite Synthese 2 so gefahren, dass die Menge an Hexen, die in der ersten Synthese 1 gebildet wird, in der zweiten Synthese 2 wieder verbraucht wird. Damit wird ein Aufbau von Hexen in der Anlage vermieden. Für den Fall, dass Hexen extern nachgefragt und deswegen ausgehend vom Teiler 9 ausgeschleust wird, wird der zweiten Synthese 2 weniger Hexen zur Verfügung gestellt, sodass auch weniger Octen gebildet werden kann. Insoweit besteht eine Konkurrenz zwischen C6 und C8-Ausbeute des Gesamtprozesses. Das Besondere des erfindungsgemäßen Verfahrens ist aber, dass es variabel gefahren werden kann zwischen einer neutralen und positiven Hexen-Bilanz und dementsprechend neben Buten und Octen zusätzlich auch Hexen als Zielprodukt anbieten kann.

Das aus der zweiten Synthese abgezogene zweite Reaktionsgemisch C2, C4, C6, C8, C8+ umfasst dieselben Olefine wie das erste Reaktionsgemisch, jedoch in anderer Zusammensetzung. Das inerte Lösemittel SOLV ist in dem zweiten Reaktionsgemisch nicht enthalten. Die zweite Synthese 2 bildet schwerpunktmäßig (neben Buten) das Octen, sodass der C8-Gehalt des zweiten Reaktionsgemisches höher ist als im ersten Reaktionsgemisch. Letzteres hat wiederum einen deutlich höheren C4-Gehalt.

Aufgrund der ähnlichen Zusammensetzung kann das zweite Reaktionsgemisch gemeinsam mit dem ersten Reaktionsgemisch aufgearbeitet werden. Dazu wird das erste Reaktionsgemisch mit dem zweiten Reaktionsgemisch an einem dritten Mischer 10 vermischt, sodass ein auf dem ersten Reaktionsgemisch basierender Stoffstrom entsteht, der in die Kolonnenserie 5, 6, 7, 8 hereingefahren wird.

Vom Kopf der vierten und letzten Destillationskolonne 8 der Kolonnenserie 5, 6, 7, 8 wird Octen C8 als zweites Zielprodukt abgezogen, im Sumpf verbleiben die höheren Olefine C8+, die als unvermeidliches Nebenprodukt separat verwertet werden.

Noch einmal zurück zu dem ersten Zielprodukt Buten C4, welches am Kopf der zweiten Kolonne 6 als $C_4$-Fraktion anfällt.

Das dort erhaltene Buten C4 ist nicht isomerenrein, sondern stellt vielmehr ein Isomerengemisch 1B, 2B aus 1-Buten und cis-2-Buten und trans-2-Buten dar. Mithin handelt es sich bei dem Kopfprodukt der zweiten Destillationskolonne 6 um lineares n-Buten. Erfreulich ist, dass es kein verzweigtes Isobuten enthält, da dieses in der ersten Synthese nicht gebildet wird. Eine aufwändige Isobuten-Abtrennung, die bei der Gewinnung von n-Buten aus C4-Strömen erforderlich ist, kann damit bei diesem auf Ethen basierenden Prozess entfallen.

Die Wirtschaftlichkeit des Prozesses kann gesteigert werden, indem das Butengemisch C4 vom Kopf der zweiten Kolonne 6 in Richtung 1-Buten weiter aufgearbeitet wird. Dafür machen die

Figuren 2, 3 und 4 jeweils einen Vorschlag.

Gemeinsames Merkmal dieser drei Varianten ist eine fünfte Destillationskolonne 11, die zur destillativen Trennung von 1-Buten 1B und 2-Buten 2B bestimmt ist. 1-Buten 1B hat einen niedrigeren Siedepunkt als cis-2-Buten und trans-2-Buten und kann daher hochrein vom Kopf der fünften Destillationskolonne 1 abgezogen werden. Für die Verwendung des 2-Butens 2B vom Sumpf der fünften Destillationskolonne 11 gibt es nun drei Möglichkeiten:

In der ersten Variante gemäß Figur 2 wird das 2-Buten einer Isomerisierung 12 unterworfen, die das 2-Buten teilweise in 1-Buten umsetzt. Nach der Isomerisierung 12 liegt wieder ein Isomerengemisch 1B, 2B aus 1-Buten und 2-Buten vor, das mit der C4-Fraktion vermischt und der fünften Destillationskolonne 11 wieder zugeführt wird. Aus thermodynamischen Gründen kann die Isomerisierung des 2-Butens nie vollständig sein. Deswegen ist es erforderlich, stetig 2-Buten 2B vom Sumpf der fünften Destillationskolonne 11 auszuschleusen.

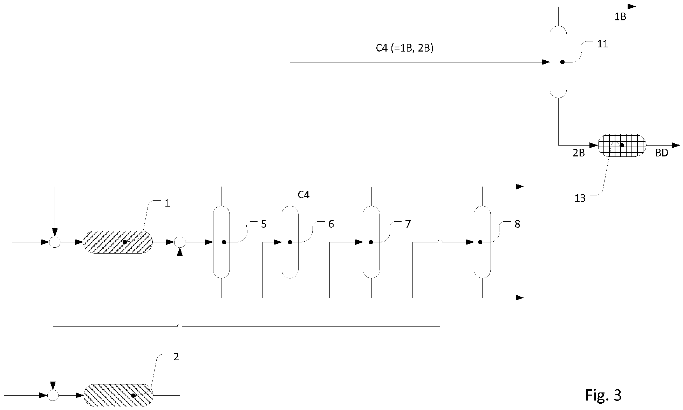

Alternativ kann das 2-Buten 2B vom Sumpf der fünften Destillationskolonne 11 einer oxidativen Dehydrierung 13 unterworfen werden. Dies ist in Figur 3 gezeigt. In der oxidativen Dehydrierung 13 wird das 2-Buten zu 1,3-Butadien BD umgesetzt, eine Chemikalie mit größerer Wertschöpfung als 2-Buten, sodass der Prozess ein viertes Zielprodukt ermöglicht. Der oxidativen Dehydrierung kann auch eine Isomerisierung von 2-Buten nach 1-Buten vorangehen, da 1-Buten schneller zu Butadien reagiert als 2-Buten. Die fakultative Isomerisierung ist in Figur 3 nicht dargestellt.

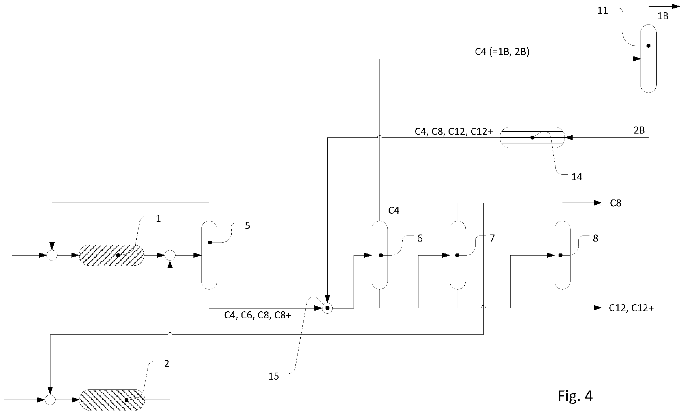

Schließlich kann gemäß Figur 4 das 2-Buten 2B vom Sumpf der fünften Destillationskolonne 11 einer dritten Synthese 14 zugeführt werden, in der es zumindest teilweise zu Octen oligomerisiert wird. Dies geschieht bevorzugt in einem OCTOL®-Prozess, der neben dem Dibuten noch Olefine mit zwölf und mehr Kohlenstoffatomen C12, C12+ bildet. Das auf diese Weise erhaltene dritte Reaktionsgemisch C4, C8, C12, C12+ wird mit dem lösemittelfreien Sumpf C4, C6, C8 und C8+ der ersten Destillationskolonne 5 in einem vierten Mischer 15 vermischt und der zweiten Destillationskolonne 6 zugeführt. Mithin erfolgt die Aufarbeitung des dritten Reaktionsgemisches gemeinsam mit dem ersten und dem zweiten Reaktionsgemisch. Die in der dritten Synthese 14 gebildeten höheren Olefine C12, C12+ geraten in den Sumpf der vierten Destillationskolonne 8 und werden dort ausgeschleust oder von dort an weiter aufgearbeitet.

**Beispiel 1: Oligomerisierung von Ethen in Isobutan**

15.5 g eines heterogenen Katalysators basierend auf Nickel und Silica-Alumina (vgl. US 2581228) wurden in einen außen mit Öl temperierten Rohrreaktor von 1 m Länge und 6 mm Innendurchmesser gefüllt. Anschließend wurde ein Gemisch aus 14.5 Massen-% Ethen und 85.5 Massen-% Isobutan mit einem Gesamtmengenstrom von 98 g/h bei einer Temperatur von 70 °C gefahren (WHSV = 6.3). Der Druck wurde auf 30 bar konstant gehalten. Nach einer Zeit von etwa 60 h war ein Zustand erreicht, in dem sich der Umsatz nicht mehr änderte. Die Ergebnisse sind in Tabelle 1 zusammengefasst. Zur weiteren Analyse wurde die Produktfraktion in einen hydrierenden Gaschromatographen gespritzt. Die Zusammensetzungen der hydrierten C8-Fraktion sind ebenfalls in Tabelle 1 zusammengefasst.

**Beispiel 2: Oligomerisierung von Ethen in und mit n-Hexen**

Analog zu Beispiel 1 wurden 15.5 g des gleichen Katalysators in einen außen mit Öl temperierten Rohrreaktor von 1 m Länge und 6 mm Innendurchmesser gefüllt. Anschließend wurde ein Gemisch aus 20 Massen-% Ethen, 73 Massen-% n-Hexen und 7 Massen-% des internen Standards n-Heptan mit einem Gesamtmengenstrom von 105 g/h bei einer Temperatur von 70 °C gefahren (WHSV = 6.8/h). Der Druck wurde auf 30 bar konstant gehalten. Nach einer Zeit von 73 h war ein Zustand erreicht, in dem sich der Umsatz nicht mehr änderte. Die Ergebnisse sowie die Zusammensetzung der hydrierten C8-Fraktion sind in Tabelle 1 zusammengefasst.

Tabelle 1: Versuchsergebnisse

| Beispiel | Umsatz | C4 | C6 | C8 | C8+ | 1-Buten | 2-Buten | Sel. nO | Sel. MH | Sel. DMH |
|---|---|---|---|---|---|---|---|---|---|---|
| 1 | 97% | 67% | 28% | 5% | 1% | 28% | 72% | 47% | 47% | 5%[a] |
| 2 | 98% | 58% | 8%[c] | 25% | 9% | 45% | 55% | 30% | 68% | 2%[b] |

Der Isoindex des C8-Gemisches beträgt für a) 0.57 und für b) 0.72.

c) C6 resultiert aus neu gebildetem Hexen abzüglich des verbrauchten Hexens.

Die Abkürzung Sel. steht für Selektivität. nO steht für n-Octen, MH für Methylhexen und DMH für Dimetyhlhexen.

**Fazit**

Beispiel 1 zeigt, dass die Verwendung eines inerten Lösemittels in der Ethen-Oligomerisierung zur Bildung von vergleichsweise mehr Buten und deutlich mehr Hexen als in Beispiel 2 und dafür kaum Octen führt. (Dieser Effekt sowie die 1-Buten-Selektivität ließen sich durch noch höhere C2-Konzentrationen verstärken.) Gleichzeitig wird in dem inerten Lösemittel ein Octen mit einem deutlich niedrigeren Isoindex gebildet als bei der Reaktion des Ethens mit Hexen, was insbesondere aus der Umsetzung von dem in dem C6-Gemisch dominierenden thermodynamisch stabileren 2- und 3-Hexen und damit dem verstärkten Aufbau eines einfach verzweigten Octens in Beispiel 2 resultiert. Wie erwünscht lässt sich durch Verwendung von Hexen als Lösemittel in der zweiten Synthese der C8-Anteil auf Kosten des C6-Anteils sichtlich erhöhen.

**Bezugzeichenliste**

| | |
|---|---|
| 1 | erste Synthese |
| 2 | zweite Synthese |
| 3 | Mischer der ersten Synthese |
| 4 | Mischer der zweiten Synthese |
| 5 | erste Destillationskolonne |
| 6 | zweite Destillationskolonne |
| 7 | dritte Destillationskolonne |
| 8 | vierte Destillationskolonne |
| 9 | Teiler |
| 10 | dritter Mischer |
| 11 | fünfte Destillationskolonne |
| 12 | Isomerisierung |
| 13 | oxidative Dehydrierung |
| 14 | dritte Synthese |
| 15 | vierter Mischer |
| C2 | Ethen |
| C4 | Buten |
| C6 | Hexen |
| C8 | Octen |
| C8+ | höhere Oligomere |
| C12, C12+ | Olefine mit zwölf und mehr Kohlenstoffatomen |
| SOLV | inertes Lösemittel |
| C2, SOLV | erstes Einsatzgemisch |
| C2, SOLV, C4, C6, C8, C8+ | erstes Reaktionsgemisch |
| [C2, SOLV]* | Leichtsiederfraktion |
| C2, C6 | zweites Einsatgemisch |
| C2, C4, C6, C8, C8+ | zweites Reaktionsgemisch |
| 1B | 1-Buten |
| 2B | 2-Buten |
| BD | Butadien |
| C4, C8, C12, C12+ | drittes Reaktionsgemisch |

**Patentansprüche**

**1.** Verfahren zur kombinierten Herstellung von zumindest Buten und Octen aus Ethen mit den folgenden Schritten:

a) Bereitstellen eines inerten Lösemittels, dessen Siedepunkt bzw. Siedebereich unterhalb der Siedepunkte der Butene liegt;
b) Bereitstellen eines ersten Einsatzgemisches enthaltend zumindest das inerte Lösemittel und darin gelöstes Ethen;
c) Überführen des ersten Einsatzgemisches in eine erste Synthese;
d) in der ersten Synthese Oligomerisieren zumindest eines Teils des im ersten Einsatzgemisch enthaltenden Ethens in Gegenwart eines ersten heterogenen Katalysators und in Gegenwart des inerten Lösemittels unter Erhalt eines ersten Reaktionsgemisches umfassend zumindest das inerte Lösemittel, Buten, Hexen und Octen;
e) Aufarbeiten des ersten Reaktionsgemisches und/oder eines darauf basierenden Stoffstroms in eine Leichtsiederfraktion enthaltend das inerte Lösemittel, mindestens eine $C_4$-Fraktion enthaltend die Butene, eine

C$_6$-Fraktion enthaltend die Hexene, eine C$_8$-Fraktion enthaltend die Octene und in eine C$_{8+}$-Fraktion enthaltend Kohlenwasserstoffe mit mehr als acht Kohlenstoffatomen;

f) Verwenden zumindest eines Teils der Leichtsiederfraktion beim Bereitstellen des ersten Einsatzgemisches;

g) Bereitstellen eines zweiten Einsatzgemisches enthaltend zumindest Hexen sowie im Hexen gelöstes Ethen unter Verwendung zumindest eines Teils der C$_6$-Fraktion;

h) Überführen des zweiten Einsatzgemisches in eine zweite Synthese, wobei die zweite Synthese von der ersten Synthese räumlich getrennt ist;

i) in der zweiten Synthese Umsetzen zumindest eines Teils des im zweiten Einsatzgemisch enthaltenden Ethens mit zumindest einem Teil des im zweiten Einsatzgemisch enthaltenden Hexens in Gegenwart eines zweiten heterogenen Katalysators unter Erhalt eines zweiten Reaktionsgemisches umfassend zumindest Octen;

k) Gemeinsame Aufarbeitung des zweiten Reaktionsgemisches mit dem ersten Reaktionsgemisch bzw. mit dem darauf basierenden Stoffstrom.

2. Verfahren nach Anspruch 1, **dadurch gekennzeichnet, dass** mit Hilfe einer ersten Destillationskolonne die Leichtsiederfraktion über Kopf aus dem ersten Reaktionsgemisch bzw. aus dem auf dem ersten Reaktionsgemisch basierenden Stoffstrom abgetrennt wird unter Erhalt eines im Wesentlichen lösemittelfreien Sumpfprodukts, aus welchem wiederum die C$_4$-Fraktion, die C$_6$-Fraktion, die C$_8$-Fraktion und die C$_{8+}$-Fraktion abgetrennt werden.

3. Verfahren nach Anspruch 2, **dadurch gekennzeichnet, dass** die Leichtsiederfraktion neben dem inerten Lösemittel in der ersten Synthese und/oder in der zweiten Synthese nicht umgesetztes Ethen enthält.

4. Verfahren nach Anspruch 1 oder nach einem der Ansprüche 2 oder 3, **dadurch gekennzeichnet, dass** es sich bei dem inerten Lösemittel um Propan oder um iso-Butan oder um ein Gemisch dieser beiden Alkane handelt.

5. Verfahren nach Anspruch 1 oder nach einem der Ansprüche 2 bis 4, **dadurch gekennzeichnet, dass** die Reaktionsbedingungen in der ersten Synthese wie folgt gewählt sind:

| | |
|---|---|
| Temperatur: | 20°C bis 150°C |
| Druck: | $1*10^5$ Pa bis $50*10^5$ Pa |
| Raum/Zeit-Belastung: | 3 bis 50 h$^{-1}$ |

und dass das molare Verhältnis von Ethen zu Lösemittel in dem ersten Einsatzgemisch eingestellt ist auf einen Wert zwischen 0.1 und 0.5, wobei der Anteil des Ethens an dem ersten Einsatzgemisch und die Reaktionsbedingungen der ersten Synthese so gewählt sind, dass das Lösemittel in einer flüssigen Phase vorliegt.

6. Verfahren nach Anspruch 5, wobei der Anteil des Ethens an dem ersten Einsatzgemisch und die Reaktionsbedingungen der ersten Synthese so gewählt sind, dass das Ethen vollständig in dem flüssigen Lösemittel gelöst ist.

7. Verfahren nach Anspruch 5, wobei der Anteil des Ethens an dem ersten Einsatzgemisch und die Reaktionsbedingungen der ersten Synthese so gewählt sind, dass das Ethen teilweise in dem flüssigen Lösemittel gelöst ist und teilweise in einer Gasphase vorliegt.

8. Verfahren nach Anspruch 1 oder nach einem der Ansprüche 2 bis 7, **dadurch gekennzeichnet, dass** der Anteil des Ethens an dem zweiten Einsatzgemisch zwischen 0.1 Gew.-% und 30 Gew.-%, beträgt, und dass die zweite Synthese bei einer Temperatur zwischen 20°C und 150 °C und bei einem Druck zwischen $1*10^5$ Pa und $50*10^5$ Pa erfolgt, wobei der Anteil des Ethens an dem zweiten Einsatzgemisch und die Reaktionsbedingungen der zweiten Synthese so gewählt sind, dass das Hexen in einer flüssigen Phase vorliegt und das Ethen darin vollständig gelöst ist.

9. Verfahren nach Anspruch 1 oder einem der Ansprüche 2 bis 8, **dadurch gekennzeichnet, dass** das zweite Reaktionsgemisch ausschließlich unter Verwendung zumindest eines Teils der C$_6$-Fraktion und unter Zugabe von Ethen bereitgestellt wird.

10. Verfahren nach Anspruch 9, **dadurch gekennzeichnet, dass** das zweite Reaktionsgemisch ausschließlich unter Verwendung eines ersten Teils der C$_6$-Fraktion und unter Zugabe von Ethen bereitgestellt wird und dass ein zweiter Teil der C$_6$-Fraktion ausgeschleust wird.

11. Verfahren nach Anspruch 9, **dadurch gekennzeichnet, dass** das zweite Reaktionsgemisch ausschließlich unter

Verwendung der gesamten $C_6$-Fraktion und unter Zugabe von Ethen bereitgestellt wird.

12. Verfahren nach Anspruch 1 oder einem der Ansprüche 2 bis 11, **dadurch gekennzeichnet, dass** als heterogener Katalysator ein Festkörper eingesetzt wird, welcher mindestens zwei Komponenten enthält, wobei die erste Komponente mindestens ein aus Ni, Cr, Fe, Ti ausgewähltes Element umfasst, welches in metallischer und/oder oxidischer und/oder hydridischer Form vorliegt, und wobei die zweite Komponente mindestens ein aus $Al_2O_3$, $SiO_2$, $TiO_2$, $ZrO_2$ ausgewähltes Metalloxid umfasst.

**Claims**

1. Process for the combined preparation of at least butene and octene from ethene with the following steps:

   a) providing an inert solvent whose boiling point or boiling range is below the boiling points of the butenes;
   b) providing a first feed mixture comprising at least the inert solvent and ethene dissolved therein;
   c) transferring the first feed mixture to a first synthesis;
   d) in the first synthesis oligomerizing at least a portion of the ethene present in the first feed mixture in the presence of a first heterogeneous catalyst and in the presence of the inert solvent to obtain a first reaction mixture comprising at least the inert solvent, butene, hexene and octene;
   e) work-up of the first reaction mixture and/or a substance stream based thereon into a low boiler fraction comprising the inert solvent, at least one $C_4$-fraction comprising butenes, a $C_6$-fraction comprising hexenes, a $C_8$-fraction comprising octenes and into a $C_{8+}$-fraction comprising hydrocarbons having more than eight carbon atoms;
   f) using at least a portion of the low boiler fraction for providing the first feed mixture;
   g) providing a second feed mixture comprising at least hexene and ethene dissolved in the hexene using at least a portion of the $C_6$-fraction;
   h) transferring the second feed mixture to a second synthesis, wherein the second synthesis is spatially separated from the first synthesis;
   i) in the second synthesis reacting at least a portion of the ethene present in the second feed mixture with at least a portion of the hexene present in the second feed mixture in the presence of a second heterogeneous catalyst to obtain a second reaction mixture comprising at least octene;
   k) joint work-up of the second reaction mixture with the first reaction mixture or with the substance stream based thereon.

2. Process according to Claim 1, **characterized in that** the low boiler fraction is separated at the top from the first reaction mixture or from the substance stream based on the first reaction mixture using a first distillation column to obtain an essentially solvent-free bottom product from which in turn the $C_4$-fraction, the $C_6$-fraction, the $C_8$-fraction and the $C_{8+}$-fraction are separated.

3. Process according to Claim 2, **characterized in that** the low boiler fraction in the first synthesis and/or in the second synthesis comprises unreacted ethene in addition to the inert solvent.

4. Process according to Claim 1 or according to either of Claims 2 or 3, **characterized in that** the inert solvent is propane or isobutane or a mixture of these two alkanes.

5. Process according to Claim 1 or according to any of Claims 2 to 4, **characterized in that** the reaction conditions in the first synthesis are selected as follows:

| | |
|---|---|
| Temperature: | 20°C to 150°C |
| Pressure: | $1*10^5$ Pa to $50*10^5$ Pa |
| Weight hourly space velocity: | 3 to 50 h$^{-1}$ |

and that the molar ratio of ethene to solvent in the first feed mixture is adjusted to a value between 0.1 and 0.5, where the proportion of ethene in the first feed mixture and the reaction conditions of the first synthesis are selected so that the solvent is present in a liquid phase.

6. Process according to Claim 5, wherein the proportion of ethene in the first feed mixture and the reaction conditions of the first synthesis are selected so that the ethene is completely dissolved in the liquid solvent.

7. Process according to Claim 5, wherein the proportion of ethene in the first feed mixture and the reaction conditions of the first synthesis are selected so that the ethene is partially dissolved in the liquid solvent and is present partially in the gas phase.

8. Process according to Claim 1 or according to any of Claims 2 to 7, **characterized in that** the proportion of ethene in the second feed mixture is in the range from 0.1% by weight to 30% by weight and **in that** the second synthesis is carried out at a temperature in the range from 20°C to 150°C and at a pressure in the range from $1*10^5$ Pa to $50*10^5$ Pa, wherein the proportion of ethene in the second feed mixture and the reaction conditions of the second synthesis are selected so that the hexene is present in a liquid phase and the ethene is completely dissolved therein.

9. Process according to Claim 1 or any of Claims 2 to 8, **characterized in that** the second reaction mixture is provided exclusively using at least a portion of the $C_6$-fraction and by adding ethene.

10. Process according to Claim 9, **characterized in that** the second reaction mixture is provided exclusively using a first portion of the $C_6$-fraction and by adding ethene and that a second portion of the $C_6$-fraction is discharged.

11. Process according to Claim 9, **characterized in that** the second reaction mixture is provided exclusively using the whole $C_6$-fraction and by adding ethene.

12. Process according to Claim 1 or any of Claims 2 to 11, **characterized in that** a solid is used as heterogeneous catalyst which contains at least two components, wherein the first component comprises at least one element selected from Ni, Cr, Fe, Ti, which is present in metallic and/or oxidic and/or hydridic form, and wherein the second component comprises at least one metal oxide selected from $Al_2O_3$, $SiO_2$, $TiO_2$, $ZrO_2$.

**Revendications**

1. Procédé de fabrication combinée d'au moins du butène et de l'octène à partir d'éthène, comprenant les étapes suivantes :

a) la préparation d'un solvant inerte, dont le point d'ébullition ou la plage d'ébullition se situe en dessous des points d'ébullition des butènes ;
b) la préparation d'un premier mélange de départ contenant au moins le solvant inerte et l'éthène dissous dans celui-ci ;
c) le transfert du premier mélange de départ dans une première synthèse ;
d) dans la première synthèse, l'oligomérisation d'au moins une partie de l'éthène contenu dans le premier mélange de départ en présence d'un premier catalyseur hétérogène et en présence du solvant inerte pour obtenir un premier mélange de réaction comprenant au moins le solvant inerte, du butène, de l'hexène et de l'octène ;
e) le traitement du premier mélange réactionnel et/ou d'un courant de matière à base de celui-ci en une fraction de composants de point d'ébullition faible contenant le solvant inerte, au moins une fraction en $C_4$ contenant les butènes, une fraction en $C_6$ contenant les hexènes, une fraction en $C_8$ contenant les octènes et en une fraction en $C_{8+}$ contenant les hydrocarbures de plus de huit atomes de carbone ;
f) l'utilisation d'au moins une partie de la fraction de composants de point d'ébullition faible lors de la préparation du premier mélange de départ ;
g) la préparation d'un deuxième mélange de départ contenant au moins de l'hexène et de l'éthène dissous dans l'hexène en utilisant au moins une partie de la fraction en $C_6$ ;
h) le transfert du deuxième mélange de départ dans une deuxième synthèse, la deuxième synthèse étant séparée dans l'espace de la première synthèse ;
i) dans la deuxième synthèse, la mise en réaction d'au moins une partie de l'éthène contenu dans le deuxième mélange de départ avec au moins une partie de l'hexène contenu dans le deuxième mélange de départ en présence d'un deuxième catalyseur hétérogène pour obtenir un deuxième mélange réactionnel comprenant au moins de l'octène ;
k) le traitement commun du deuxième mélange réactionnel avec le premier mélange réactionnel ou avec le courant de matière à base de celui-ci.

**2.** Procédé selon la revendication 1, **caractérisé en ce qu'**à l'aide d'une première colonne de distillation, la fraction de composants de point d'ébullition faible est séparée par la tête à partir du premier mélange réactionnel ou à partir du courant de matière à base du premier mélange réactionnel pour obtenir un produit de fond essentiellement exempt de solvant, à partir duquel la fraction en $C_4$, la fraction en $C_6$, la fraction en $C_8$ et la fraction en $C_{8+}$ sont séparées.

**3.** Procédé selon la revendication 2, **caractérisé en ce que** la fraction de composants de point d'ébullition faible contient en plus du solvant inerte de l'éthène non réagi dans la première synthèse et/ou dans la deuxième synthèse.

**4.** Procédé selon la revendication 1 ou selon l'une quelconque des revendications 2 ou 3, **caractérisé en ce que** le solvant inerte est le propane ou l'isobutane ou un mélange de ces deux alcanes.

**5.** Procédé selon la revendication 1 ou selon l'une quelconque des revendications 2 à 4, **caractérisé en ce que** les conditions de réaction dans la première synthèse sont choisies tel que suit :

| | |
|---|---|
| température : | 20 °C à 150 °C |
| pression : | $1*10^5$ Pa à $50*10^5$ Pa |
| chargement espace/temps : | 3 à 50 $h^{-1}$ |

et **en ce que** le rapport molaire entre l'éthène et le solvant dans le premier mélange de départ est ajusté à une valeur comprise entre 0,1 et 0,5, la proportion d'éthène dans le premier mélange de départ et les conditions de réaction de la première synthèse étant choisies de sorte que le solvant se présente dans une phase liquide.

**6.** Procédé selon la revendication 5, dans lequel la proportion d'éthène dans le premier mélange de départ et les conditions de réaction de la première synthèse sont choisies de sorte que l'éthène soit entièrement dissous dans le solvant liquide.

**7.** Procédé selon la revendication 5, dans lequel la proportion d'éthène dans le premier mélange de départ et les conditions de réaction de la première synthèse sont choisies de sorte que l'éthène soit partiellement dissous dans le solvant liquide et partiellement présent dans une phase gazeuse.

**8.** Procédé selon la revendication 1 ou selon l'une quelconque des revendications 2 à 7, **caractérisé en ce que** la proportion d'éthène dans le deuxième mélange de départ est comprise entre 0,1 % en poids et 30 % en poids, et **en ce que** la deuxième synthèse a lieu à une température comprise entre 20 °C et 150 °C et à une pression comprise entre $1*10^5$ Pa et $50*10^5$ Pa, la proportion d'éthène dans le deuxième mélange de départ et les conditions de réaction de la deuxième synthèse étant choisies de sorte que l'hexène se présente dans une phase liquide et que l'éthène soit entièrement dissous dans celui-ci.

**9.** Procédé selon la revendication 1 ou selon l'une quelconque des revendications 2 à 8, **caractérisé en ce que** le deuxième mélange réactionnel est préparé exclusivement en utilisant au moins une partie de la fraction en $C_6$ et avec ajout d'éthène.

**10.** Procédé selon la revendication 9, **caractérisé en ce que** le deuxième mélange réactionnel est préparé exclusivement en utilisant une première partie de la fraction en $C_6$ et avec ajout d'éthène, et **en ce qu'**une deuxième partie de la fraction en $C_6$ est mise au rebut.

**11.** Procédé selon la revendication 9, **caractérisé en ce que** le deuxième mélange réactionnel est préparé exclusivement en utilisant l'ensemble de la fraction en $C_6$ et avec ajout d'éthène.

**12.** Procédé selon la revendication 1 ou selon l'une quelconque des revendications 2 à 11, **caractérisé en ce qu'**un corps solide est utilisé en tant que catalyseur hétérogène, qui contient au moins deux composants, le premier composant comprenant au moins un élément choisi parmi Ni, Cr, Fe, Ti, qui se présente sous forme métallique et/ou oxydique et/ou hydrurique, et le deuxième composant comprenant au moins un oxyde métallique choisi parmi $Al_2O_3$, $SiO_2$, $TiO_2$, $ZrO_2$.

Fig. 1

Fig. 2

1B

C4 (=1B, 2B)

11

2B

BD

13

C4

1

5

6

7

8

2

Fig. 3

Fig. 4

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102008007081 A1
- EP 1029839 A1
- EP 2582648 B1
- WO 2005123884 A
- WO 2005123633 A
- US 20130066128 A1
- US 8637722 B2
- WO 2010117539 A1
- US 4717782 A
- US 8637722 B
- US 20130158321 A1
- US 2581228 A

**In der Beschreibung aufgeführte Nicht-Patentliteratur**

- Butenes. **F. GEILEN ; G. STOCHNIOL ; S. PEITZ ; E. SCHULTE-KOERNE.** Ullmann's Encyclopedia of Industrial Chemistry. 2013
- **B. SCHOLZ.** The HÜLS OCTOL Process: Heterogeneously catalyzed dimerization of n-butenes and other olefins. *DGMK-Tagung,* April 1989, 21, , 22
- **R.H. FRIEDLANDER ; D.J. WARD ; F. OBENAUS ; F. NIERLICH ; J. NEUMEISTER.** Make plasticizer olefins via n-butene dimerization. *Hydrocarbon Processing,* Februar 1986, 31-33
- **F. NIERLICH.** Oligomerize for better gasoline. *Hydrocarbon Processing,* Februar 1992, 45-46